# EUROPEAN PATENT SPECIFICATION

(11) **EP 0 531 447 B1**
(45) Date of publication and mention of the grant of the patent: **10.11.1999**
(21) Application number: 91911934.7
(22) Date of filing: 22.05.1991
(51) Int. Cl.: A61K 31/70, C07H 21/04, C12Q 1/68

(54) **COMPOSITIONS AND METHODS FOR MODULATING RNA ACTIVITY THROUGH MODIFICATION OF THE 5' CAP STRUCTURE OF RNA**
ZUSAMMENSETZUNGEN UND VERFAHREN ZUR REGULIERUNG DER RNS-AKTIVITÄT DURCH ABÄNDERUNG DER RNS "5'-CAP-STRUKTUR"
COMPOSITIONS ET PROCEDES SERVANT A MODULER L'ACTIVITE DE L'ARN PAR MODIFICATION DE LA STRUCTURE 5' CAP DE L'ARN

(30) Priority: 23.05.1990 US 527599
(43) Date of publication of application: 17.03.1993
(73) Proprietor: ISIS PHARMACEUTICALS, INC., Carlsbad, CA 92008 (US)
(72) Inventor: BAKER, Brenda, F., Carlsbad, CA 92009 (US)
(74) Representative: Hallybone, Huw George
(86) International application number: US9103606
(87) International publication number: WO9117755

(56) References cited:
- WO-A-87/07300
- WO-A-91/10671
- GENE vol. 72, no. 1-2, 10 December 1988, AMSTERDAM NL pages 51 - 58 TOULM , J.-J. & H L NE, C. 'Antimessenger oligodeoxyribonucleotides: an alternative to antisense RNA for artificial regulation of gene expression - a review'
- JOURNAL OF THE NATIONAL CANCER INSTITUTE vol. 81, no. 20, 18 October 1989, pages 1539 - 1545 ROTHENBERG, M. ET AL. 'Oligodeoxyribonucleotides as anti-sense inhibitors of gene expression: therapeutic implications'
- SCIENCE vol. 240, 22 April 1988, LANCASTER, PA US pages 426 - 433 SCHULTZ, P.G. 'The interplay between chemistry and biology in the design of enzymatic catalysts'
- PROCEEDINGS OF THE NATIONAL ACADEMY OF SCIENCES OF USA vol. 85, August 1988, WASHINGTON US pages 5507 - 5511 GOODCHILD, J. ET AL. 'Inhibition of human immunodeficiency virus replication by antisense oligodeoxynucleotides'
- FEBS Letters, Volume 96, Number 1, issued December 1978, W. FILIPOWICZ, "Functions of the 5'-Terminal m7G CAP in Eukaryotic mRNA", pages 1-11, see pages 1-11.
- Nucleic Acids Research, Volume 15, No. 21, issued 1987, LE DOAN et al., "Sequence-Targeted chemical modifications of Nucleic Acids by complementary oligo-nucleotides covalently linked to porphyrins", pages 8643-8659, see pages 8643-8659.

## Description

### FIELD OF THE INVENTION

This invention is directed to compositions for achieving therapeutic treatment of diseases and for regulating gene expression in biological experimental systems. The invention generally relates to the field of "antisense" compounds in conjunction with the field of gene expression, especially protein expression, which is dependent upon the 5' Cap of eucaryotic and viral RNAs.

### BACKGROUND OF TEE INVENTION

Antisense compounds are molecules that are capable of hybridization with a specific nucleotide sequence of an RNA. The 5' Cap of eucaryotic and viral RNAs is a structurally and chemically unique entity located at the 5' terminus of RNAs and is a determinant for the maturation, lifetime and efficacy of translation of the RNA.

It is well known that most of the bodily functions in mammals including most disease states, are effected by proteins. Such proteins, either acting directly or through their enzymatic functions, contribute in major proportion to many diseases in animals and man. Classical therapeutics have generally focused upon interactions with such proteins in efforts to moderate their disease causing or disease potentiating functions.

Recently, however, attempts have been made to selectively moderate the actual production of such undesired proteins by interactions with molecules that direct their synthesis, intracellular RNA. These interactions involve the binding of complementary "antisense" oligonucleotides or their analogs to the intracellular RNA in a sequence specific fashion by Watson-Crick base pairing interactions. Intracellular hybridization of the two molecules is intended to inhibit either the synthesis and proper metabolism of the selected mRNA or its utilization by the translational machinery in the synthesis of proteins. It has been believed that interference with the production of proteins in this manner would yield a therapeutic effect with minimal side effects due to the high level of reaction specificity available through RNA sequence recognition by the antisense molecules. See *Cancer Res*. 48 2659-68 (1988); *Pharmaceutical Res*. 5 539-49 (1988); *Anticancer Drug Design* 2 117-128 (1987)

Based on experimental observations to date, a number of chemical modifications have been introduced into antisense oligonucleotides to increase their therapeutic activity. These modifications have been intended to increase the cell penetration of the antisense oligonucleotides, to stabilize them from nucleases and other enzymes that degrade or interfere with their structure and activity intracellularly, and to improve and increase their efficacy in the inhibition of protein production. These modifications include alteration of the backbone structure for improved stabilization and penetration into the cell, e.g., methyl phosphonates, *Nucleic Acid Research* 6 3009-24 (1979), *Biochemistry* 18 5134-43 (1979); methyl phosphorothionates, *Journal of the American Chemical Society* 111 2321-22 (1989); phosphorothioates, *Proceedings of the National Academy of Science* 85 7079-7083 (1987); and phosphoroamidates, *Biochemistry* 27 7237-46 (1986), *Nucleic Acid Research* 14 3487-99 (1986). They include the attachment of additional types of binding domains and/or reaction centers to the antisense portion to enhance the efficacy of action of the antisense molecule, e.g., intercalators, *Nucleic Acid Research* 15 4717-36 (1987), *Biochemistry* 27 3997-4003 (1988); alkylating agents; and redox metals, *Nucleic Acid Research* 15 8643-59 (1987), *Acc*. Chem. *Res* 19 180-86 (1986).

Regardless of these modifications the activity of the antisense oligonucleotides previously available has not been sufficient for practical therapeutic, research, or diagnostic use. The basis of this insufficiency is likely several fold, incomplete understanding of the secondary and tertiary structures of the targeted RNA, low percentages of delivery and uptake, inactivation of reactive centers by other cellular components, and requirements for stoichiometric conditions for inhibition of protein production.

Thus, there has been a long-felt need for oligonucleotides which are effective for therapeutic use and possessed of no or few side effects.

### OBJECTS OF THE INVENTION

It is a principal object of this invention to provide therapeutic compositions for the treatment of diseases through modulation of the activity of RNA.

It is a further object of this invention to provide oligonucleotide analogs for use in therapeutics.

A further object of this invention is to provide such oligonucleotide analogs which are less likely to invoke undesired or toxic side reactions or effects.

Another object of this invention is to provide analogs that inhibit the maturation, stabilization, and/or initiation of translation of a selected mRNA.

A still further object of this invention is to utilize antisense oligonucleotide analogs that chemically or structurally modify the 5' Cap structure of messenger RNA.

A further object of this invention is to utilize the unique structural and chemical features of the 5' Cap of messenger RNA to modify or remove it.

A further object of this invention is to catalytically remove or modify the 5' Cap of RNA.

Yet another object of this invention is to non-catalytically remove or structurally or chemically modify the 5' Cap of RNA.

These and other objects will become apparent to persons of ordinary skill in the art from a review of the present specification and attendant claims.

### SUMMARY OF THE INVENTION

In accordance with the present invention, compositions for modulating the activity of RNA are provided. These compositions comprise a reactive portion capable of chemically or structurally altering or removing the 5' Cap structure of a targeted RNA. The composition further provides a targeting portion which is specifically hybridizable with the 5' terminal region of a targeted RNA for placement of the reactive portion in a reactive position for the 5' Cap. The compositions also include a tether portion for connecting the targeting and reactive portions together.

In accordance with preferred embodiments, the reactive portion of the composition comprises one or more functionalities capable of catalytically removing or catalytically modifying the 5' Cap of messenger RNA. In other preferred embodiments, the reactive portion of the composition comprises one or more functionalities capable of chemically or structurally modifying or removing the 5' Cap of messenger RNA in a non-catalytic manner. Such functionalities may be nucleophilic, electrophilic, basic, acidic, cationic, amphoteric, or redox active for such purposes. Specific examples of such moieties include imidazole, N-methylimidazole, histamine, pyridine, 1,5,9-triazacyclododecane, diethylene triamine, triethylene tetramine, and zinc(II) or copper(II) complexes of 1,10-*ortho*-phenanthroline or bipyridine. Of these, triethylene tetramine is preferred, and the copper(II) complexes even more preferred.

In accordance with preferred embodiments the targeting portion of this invention comprises an oligonucleotide (or analog) from 5 to 50 base units which recognizes the 5' terminal region of the targeted transcript.

The targeting portion is preferably an analog of an oligonucleotide wherein at least some of the oligonucleotide has been substituted with structure which functions to enhance the ability of the compositions to penetrate into the intracellular region of cells where the RNA whose activity is to be modulated is located and to provide nuclease resistance. In accordance with one preferred embodiment, the oligonucleotides and oligonucleotide analogs are formulated such that at least some of the linking groups between nucleotide units of the oligonucleotide comprise sulfur-containing species such as phosphorothioate moieties.

The tether portion of the composition comprises functionalities which will optimize the placement of the reactive portion with respect to the targeting portion for removal or modification of the 5' Cap structure. Such functionalities may have specific hydrogen donor and acceptor capabilities and motifs for optimal placement of the reactive portion. In one embodiment the tether comprises one or more nucleotides. In another embodiment the tether comprises one or more amino acids.

The compositions of the invention can also be used for modulating the production of a protein by an organism comprising contacting the organism with a composition formulated in accordance with the foregoing considerations. It is preferred that the targeted RNA sequence be preselected to comprise that portion of RNA, preferably messenger RNA, which codes for the protein whose formation is to be modulated, inhibited or arrested. The targeting portion of the composition to be employed is, thus, selected to be complementary to or the antisense of the preselected sequence of RNA.

The compositions of the invention can also be used for treating an organism having a disease characterized by the undesired production of a protein comprising contacting the organism with a composition in accordance with the same preferable considerations as given in the previous paragraph.

### BRIEF DESCRIPTION OF THE DRAWINGS

Figure 1 depicts the structure of the 5' Cap.
Figure 2 depicts sites for attack upon the methylated guanosine residue of the 5' Cap structure of mRNA.
Figure 3 shows sites for attack upon the triphosphate linkage of the 5' Cap structure of mRNA.
Figure 4 depicts two approaches to utilization of cytosine as an appendage or anchorage to the tether to enhance placement of the reactive moiety.

### DETAILED DESCRIPTION

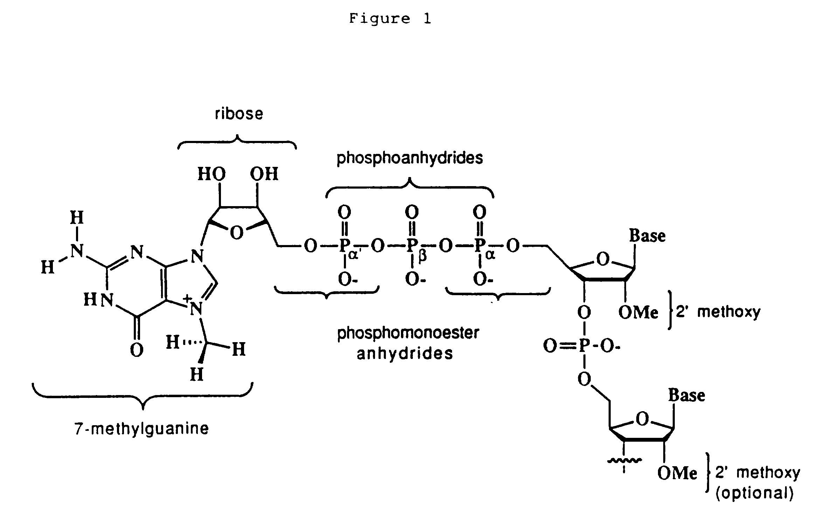

It has been recognized that the majority of eucaryotic and viral small nuclear RNAs and messenger RNAs have a unique chemical structure at their 5' terminus which is required in varying degrees for their maturation, stability, and efficacy in translation. The general structural features are given in *J. Mol. Biol*. 99 519-547 (1975) and are shown in Figure 1. The cap is a guanosine residue which is methylated at the nitrogen 7 position. It is joined to the penultimate 5' base of the RNA via a triphosphate linkage between the 5' hydroxyl groups of each residue. The 2' hydroxyls of the 5' terminal base(s) are methylated.

The difference between the caps of small nuclear RNAs and messenger RNAs is that the small nuclear RNA cap is dimethylated at the exocyclic amino group of the guanine residue. This feature is believed to be an important determinant in the functional destiny of small nuclear RNAs versus messenger RNAs, *Nucleic Acid Research* 16 8953 (1988).

The cap structure is added to nascent transcripts during transcription in the nucleus. Once transcribed, the primary transcript from eucaryotic and certain viral genes must be processed due to the presence of intervening sequences within the coded regions of the transcript. The 5' Cap has been shown to be necessary for processing of the primary transcripts to mature RNA molecules, specifically in the splicing reactions during which removal of the introns occurs. See *Cell* 38 731-736 (1984). Removal or lack of a 5' Cap results in rapid degradation of the RNA in the nucleus and the cytoplasm as described in *Mol*. *Biol. Med*. 5 1-14 (1988) and *Cell* 32 681-694 (1983). Without the 5' Cap the RNA is presumably accessible to 5' exonucleases. Finally, the majority of eucaryotic and viral mRNAs studied to date require the 5' Cap for the initiation of translation; See *Cell* 9 645-653 (1976); *Federation of Experimental Biologists Society Letter* 96 1-11(1978); and *Prog*. *Nuc. Acid Res*. 35 173-207 (1988). There are also specific Cap binding proteins which are components of the machinery required for the initiation of translation of a protein. See *Cell* 40 223-24 (1985); and *Prog. Nuc. Acid Res*. 35 173-207 (1988). Based on the current understanding of the properties and function of the 5' cap structure of messenger RNA it is now believed that certain modifications of the structure, such as removal or structural or chemical alterations, will preclude the transcript from any pertinent processes, such as the production of an undesired protein. It is now believed that oligonucleotides can provide such interference.

In general, the compositions necessary for modulating the activity of an RNA transcript in accordance with this invention may be regarded in three portions: the reactive moiety, the tether, and the antisense binding/recognition unit.

The function of the reactive moiety is to remove or alter the 5' Cap of the targeted transcript such that the transcript is unable to operate in one or more of its normal processes beginning from the time of synthesis of the targeted transcript's 5' Cap structure to the time of degradation and removal of the targeted transcript from the translation pool.

The function of the tether is to link the reactive moiety and the antisense binding portion of the composed molecule together. The tether may include organic and/or inorganic functional groups which will optimize the position and orientation of the reactive moiety to achieve the utmost precision in specific activity towards removal or modification of the 5' Cap.

The function of the antisense binding/recognition unit is to direct the reactive moiety specifically to the 5' terminal region of the targeted transcript, preferably without interference in non-targeted cellular processes and in a manner which facilitates the functional ability of the reactive moiety and tether.

Each aspect of the 5' Cap structure can be exploited singly or together as required to achieve the objectives of this invention. These include the phosphoanhydride linkages, the phosphomonoester anhydride linkages, the methylated guanine residue and its appended ribose residue. Examples of approaches for each aspect follow.

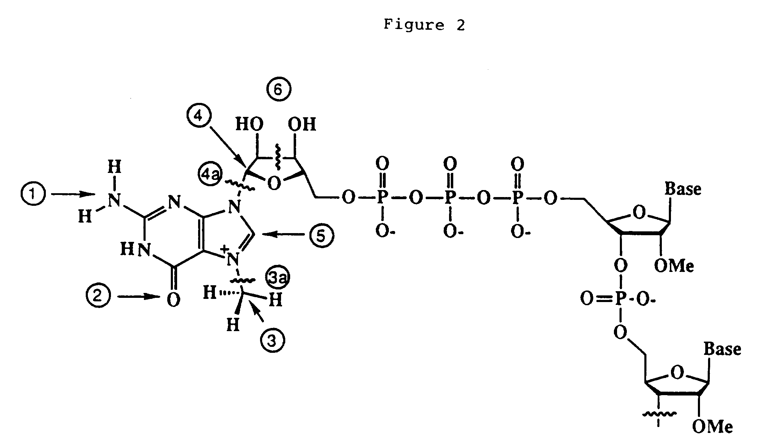

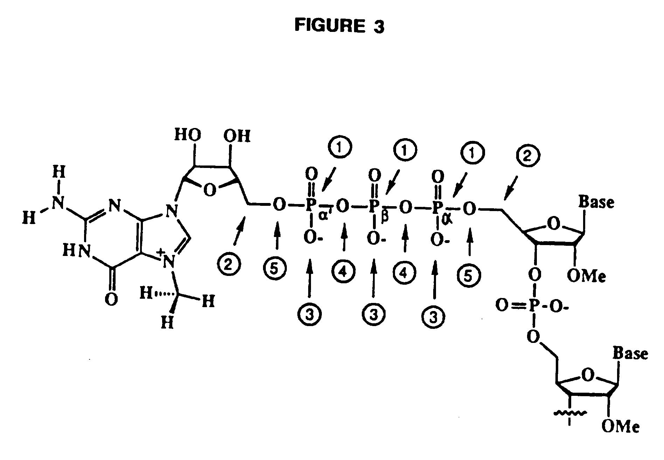

Figures 2 and 3 depict the reactive atoms and bonds of the 5' Cap structure which because of their inherent chemical nature are susceptible to modification or cleavage given the appropriate reactive moiety.

Figure 2 displays sites of the methylated guanine residue and its appended ribose residue which are viable targets for modification of the 5' Cap structure. Both the exocyclic nitrogen at the 2 position (site 1) and the oxygen at the 6 position (site 2) of the methylated base are nucleophiles. Therefore, they may be modified via alkylation utilizing functional groups such as sulfonyl alkyl halides, alpha-halo carbonyls, or aziridines.

Both the nitrogen methyl bond (site 3a) and the nitrogen-glycosidic bond (site 4a) are labile due to the electron deficient state of the aromatic ring. Therefore, nucleophilic attack at the 7-methyl carbon (site 3) or carbon one of the ribose (site 4) would result in cleavage of the nitrogen carbon bond to yield an aberrant cap structure. Reactive groups include amines, hydroxyls, and sulfhydryls.

The carbon at the 8 position of the methylated guanine residue (site 5) is electrophilic due to the methylated and consequently electropositive nitrogen at position 7. Therefore, this site is amenable to reactions with nucleophilic groups such as amines and hydroxyls.

Cleavage of the bond between carbon 3 and carbon 4 of the sugar ring (site 6) may be achieved oxidatively via the 2' and 3' hydroxyl groups utilizing reactive moieties such as chelated metals.

Figure 3 indicates those sites of the phosphate chain which are viable targets for the modification and removal of the 5' Cap structure utilizing nucleophiles and/or electrophiles as reactive groups.

Both the phosphorus atoms (site 1) and the carbon 5 atoms (site 2) are amenable to attack by a nucleophile. Nucleophilic attack would result in displacement of one of the attached intrachain oxygen atoms and thus cleavage of the phosphoanhydride chain between the penultimate base of the mRNA and the methylated guanosine. These reactions can be catalytic given the appropriate choice of nucleophile (e.g. amines and carboxylates).

The oxygen atoms at sites 3, 4, and 5 are all sites for enhancement or activation of the catalytic cleavage reactions by nucleophiles (at sites 1 and 2) via protonation or metal interactions using additional functional groups appended to the tether. In addition these oxygens are susceptible to electrophiles and thus alkylation which would result in irreversible modification of the phosphoanhydride linkage.

The reactive moiety of a composed therapeutic molecule can be a composite itself with multiple functional groups to achieve the desired reaction or simply one functional group to do the same. As exemplified previously, single entities available include nucleophiles, e.g., amines, carboxylates, thiocarboxylates, and hydroxides via coordination chemistry; Lewis or Brönsted acids and bases, including metals; redox active functional groups, e.g., chelated metals; and electrophiles, e.g., alpha-halo carbonyls/amides, aziridines, methyl transferases.

One of several plausible means for catalytic removal of the 5'Cap involves utilization of pyridine as the reactive moiety. Pyridine, acting as a nucleophile, is capable of cleaving exclusively pyrophosphate diesters in the presence of phosphodiesters under aqueous conditions, *Can. J. Biochem.* 50 287-291 (1972). This type of nucleophile, an aromatic nitrogen, cleaves the pyrophosphate bond via a covalent intermediate. The intermediate is then hydrolyzed to release the cleaved product and the reactive nucleophile. It is thus a catalytic reaction.

Pyridine can be tethered by known synthetic methodologies, *Organic Chemistry*, Vol. 19, A.R. Katritzky and J.M. Lagowski (1971), from one of several sites on its aromatic ring to an antisense oligonucleotide. Such an attachment is intended to place the reactive pyridinic nitrogen in close and reactive proximity to one of the electrophilic phosphorus or carbon atoms of the 5' Cap. As an example, attachment of a variety of substituents to the 4 position of the aromatic ring may be accomplished via the N-oxide derivative of pyridine. This compound is derived from the reaction between pyridine and peracetic acid. Reaction upon the N-oxide via either electrophilic (nitration) or nucleophilic reaction mechanisms (alcohols, amines, halogens, sulfhydryls, or organometallic groups) will generate pyridine derivatives appropriately functionalized, e.g., with an amine or a carboxylic acid, for the attachment to the antisense binding unit.

The advantage of pyridine as a nucleophile, in the case of the phosphorus atoms, is that it is neutral and thus will experience nominal electrostatic interference from the anionic phosphate oxygens, *Science* 235 1173-1178 (1987). Based on the pKa values of the nucleoside mono, di, and triphosphates and the relationship of pKa to the leaving group ability in nucleophilic displacement reactions it is believed that it is preferred to place a nucleophile in an optimal position for nucleophilic attack on the β phosphorus atom of the 5' Cap linkage.

In addition to pyridine, other organic alkyl and aromatic amines are provided which act as nucleophiles, Lewis acid/bases or general acid/bases and which cause chemical alteration of the 5' Cap. Imidazole, N-methylimidazole, histamine, 1,5,9-triazacyclododecane, diethylene triamine and triethylene tetramine were all shown to react with the 5' Cap structure; it is presently believed that the reaction of these amines upon the 5' Cap causes hydrolysis of the 7-methylguanosine residue.

In addition to the amines, metal complexes are provided that have been found to be effective in chemical removal (cleavage) of the 5' Cap. Copper(II) and zinc(II) complexes of the chelators 1,10,-*ortho*-phenanthroline and bipyridine are even more reactive upon the 5' Cap structure than are the alkyl and aromatic amines. Of these four metal complexes, the copper(II)-*ortho*phenanthroline complex was most reactive. It is presently believed that this complex reacts with the 5' Cap to cause hydrolysis of the phosphoanhydride linkage in a non-catalytic manner.

The detailed placement of the reactive moiety is governed by the tether or linker between it and the antisense binding unit. The length of the tether may be anywhere between 1 and 50 atoms, or more preferably between 1 and 500 atoms, excluding any additional appendages, e.g., functional groups appended to the main chain of the tether. Preferred means of attachment of the tether to the antisense binding unit are via ethers, esters, or amides from the sugar or phosphate residue of the penultimate base of the antisense strand. Attachments may also be directly from the penultimate base, e.g., the 5 position of the pyrimidines. Attachments may also be from the 2 position of the penultimate base in the case of purines. In one embodiment the tether comprises one or more amino acids. In another embodiment the tether comprises one or more noncomplementary natural or modified nucleotides. As shown in Example 6, the presence of two extra "dangling" bases extending off the 3' end of an antisense oligodeoxyribonucleotide (opposite the 5' Cap of the RNA) does not inhibit specific hybridization of the antisense oligonucleotide to a 5'-capped RNA.

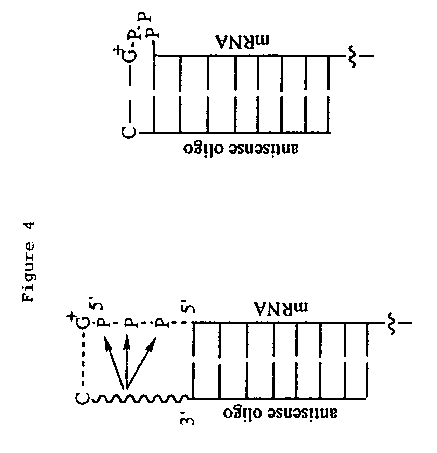

Appendages of the tether may include additional binding units, such as a cytosine residue or a guanidinium group, which are specific to the 5' Cap structure, such as the methylated guanosine residue or the anionic phosphate groups respectively. Figure 4 shows a set of examples for a cytosine appendage. The intent is to add an additional binding element to further constrain or fix the conformation of the 5' Cap. Additionally, it may provide an avenue for improved placement (or anchorage) of a catalytic cleavage moiety, such as a nucleophile, next to the alpha or beta phosphorus atoms of the cap linkage. The cytosine may be attached via a specialized tether, in length or composition, or via one of the known phosphate linkages.

The final portion of the composition, to be considered, is the antisense binding unit. This portion of the molecule is what targets the reactive moiety to the messenger RNA which has been selected for modification. In this invention, it specifically locates the antisense molecule to the 5' region of the selected transcript in reactive proximity to the 5' Cap structure. The targeting portion of the composition is generally either an oligonucleotide or oligonucleotide analog. It is designed and synthesized, generally through solid state synthesis, solution phase synthesis or enzymatic synthesis of known methodology. Nucleic acid synthesizers and relevant enzymes are commercially available; the use of which is generally understood by persons of ordinary skill in the art. The available methodologies are capable of generating nearly any oligonucleotide of reasonable length which may be desired.

In the context of this invention, the term "oligonucleotide" refers to a plurality of joined nucleotide units formed from naturally-occurring bases and furanosyl groups joined by native phosphodiester bonds. This term effectively refers to naturally-occurring species or synthetic species formed from naturally-occurring subunits.

"Oligonucleotide analog," as that term is used in connection with this invention, refers to moieties which function similarly to oligonucleotides but which have non-naturally occurring portions. Thus, oligonucleotide analogs may have altered sugar moieties or inter-sugar linkages. Exemplary among these are the phosphorothioate and other sulfur-containing species which are known for use in the art. Persons of ordinary skill in the art will be able to select other linkages for use in the practice of the invention.

Oligonucleotide analogs may also include species which include at least some modified base forms. Thus, purines and pyrimidines other than those normally found in nature may be so employed. Similarly, modifications on the furanose portions of the nucleotide subunits may also occur as long as the essential tenets of this invention are adhered to.

Such analogs are best described as being functionally interchangeable with natural oligonucleotides (or synthesized oligonucleotides along natural lines), but which have one or more differences from natural structure. All such analogs are comprehended by this invention so long as they function effectively to hybridize with the RNA molecule bearing the 5' Cap to be structurally or chemically modified.

It is preferred in some embodiments of the present invention to employ oligonucleotide analogs rather than the oligonucleotides themselves. In this context, oligonucleotide analog refers to structure which is generally similar to native oligonucleotides in its ability to complex with the sense strand. Modifications include those that enhance the ability of the antisense molecule to penetrate into the intracellular spaces of cells where the targeted messenger RNA resides and those modifications which provide nuclease resistance. For these purposes it is currently preferred to substitute modified backbones, non-ionic, non-chiral or enantiomerically pure entities, in place of some or all of the phosphodiester bonds. Modifications may also include those that enhance the attachment and/or placement of the tether and reactive moiety in order to achieve optimal reactivity with the 5' Cap. To achieve this goal might include usage of alpha-anomeric oligonucleotides which will bind parallel (5'-3':5'-3') to the 5' terminal sequence of the targeted transcript. Any of the existing means for accomplishing these goals may be employed in accordance with the practice of the present invention. The targeting portions of the compositions of the present inventions, are preferably oligonucleotides or oligonucleotide analogs having about 5 to about 50 base units, or base analogs. It is more preferred that such functionalities have about 8 to 40 base units.

While the present invention has been described with specificity in accordance with certain of its preferred embodiments, the following examples serve only to illustrate the invention and are not intended to limit the same.

### EXAMPLES

### Example 1

Cleavage of m7GpppG by metal complexes and amines: Several copper complexes, alkyl amines and aromatic amines that were judged to be good candidates for eventual tethering to antisense oligonucleotides were assayed for their ability to chemically modify m7GpppG (Pharmacia LKB Biotechnology), a single quanosine nucleotide capped with the methylated guanosine (m7G) cap structure. This structure is analogous to the 5' Cap and first (5'-most) nucleotide, here a guanosine, of an mRNA molecule.
(a) Copper complexes: Copper (II) complexes of 1,10-*ortho*-phenanthroline (purchased from Lancaster Synthesis) and bipyridine (Aldrich) were assayed as follows. Copper complexes (50-500 µM) and m7GpppG (50-500 µM) were combined in 20 mM HEPES buffer, pH 7.1, in 1.7 ml Eppendorf tubes at reaction volumes of 300 µl. Varying concentrations were used to determine the best ratio of copper complex to substrate. Reactions were carried out at 37°C for 24 hours, with centrifugation and remixing at 6-8 hour intervals.
(b) Alkyl and aromatic amines: Imidazole, N-methylimidazole, histamine, pyridine, 1,5,9-triazacyclododecane, diethylene triamine and triethylene tetramine (all purchased from Aldrich) were assayed as follows. 500mM amine and 1 mM m7GpppG were combined in 0.65 ml Eppendorf tubes at a total reaction volume of 20 µl. Reactions were carried out at 60°C for 12 hours, with centrifugation and remixing every hour to minimize concentration fluctuation due to evaporation and condensation.

### Example 2

Analysis of reactions by anion exchange chromatography: An aliquot of each reaction was removed at each specified time interval for chromatographic analysis. Injection samples were prepared by addition of the internal standard, nicotinamide adenosine diphosphate (NAD, purchased from Boehringer Mannheim), and dilution to a final volume of 110 µl with double distilled water. Prepared samples were then injected into a 100 µl injection loop and subsequently loaded onto a Pharmacia LKB FPLC (Fast Protein Liquid Chromatography) system utilizing a MonoQ HR 5/5 anion exchange column. Solvent A: distilled water. Solvent B: 1 M NaCl plus 5 mM Na Phosphate (pH 7.0). Program gradient: 0 to 40% B in 30 minutes, 40% B for 1 minute, 40% to 100% B in 1 minute, 100% B for 1 minute, 100% to 0% B in 0.1 minute, and 0% B for 10 minutes. Flow rate = 1 ml/minute. Detection of products was by UV absorption at 260 nm. Integration was performed by the internal program on the LC-500 FPLC Control panel. Relative rates for the alkyl amines and imidazoles are based upon the amount of remaining substrate, m7GpppG, measured against the internal standard, NAD, at t=0 hours and t=12 hours. Relative rates for the copper(II) complexes are based upon the amount of remaining substrate, m7GpppG, measured against the internal standard, NAD, and that of the control (buffer only) under the same reaction conditions. For UV analysis of the reactants and products, peaks were collected during the course of chromatography and then scanned separately using a photodiode array detector.

### Example 3

Reaction rates and product analysis:
Of the compounds examined, the most reactive upon the 5' Cap structure, m7GpppG, are the copper complexes. Cu(II):orthophenanthroline hydrolyzes 52% of the starting material after 24 hours at 37°C. This metal complex yields as products GMP, m7GMP, GDP and m7GDP, as shown by chromatography. Product analysis was performed by coinjection experiments utilizing commercially available standards and by UV spectral analysis. These products indicate that hydrolysis of the phosphoanhydride linkage of the substrate is occurring. Further investigation of the reaction between Cu(II):orthophenanthroline and both the asymmetric and symmetric phosphoanhydrides, m7GpppG and GpppG, showed that the ratio of metal complex to the substrate must be greater than or equal to 2:1 in order to observe hydrolysis under the times (up to 7 days), temperatures (22°C, 37°C, 60°C) and reactant conditions studied. Thus, it is presently believed that these reactions are not catalytic, since turnover was not observable.

In comparison to the metal complexes, the aromatic and alkyl amines were relatively unreactive. In order to observe these reactions within a reasonable time frame (24 hours), the temperature and concentrations were drastically increased over those used in the metal complex reactions. The relative reactivities of the amines with m7GpppG are shown in Table 1.

**TABLE 1**

| Reagent | Rel. reactivity upon m7GpppG |
|---|---|
| Triethylene tetramine | 1.0 |
| 1,5,9-Triazacyclododecane | 0.8 |
| Histamine | 0.24 |
| Diethylene triamine | 0.10 |
| Imidazole | 0.05 |
| N-methylimidazole | 0.05 |
| Pyridine | 0.01 |

Of the amines assessed (imidazole, N-methylimidazole, histamine, pyridine, 1,5,9-triazacyclododecane, diethylene triamine, and triethylene tetramine), triethylene tetramine was most reactive, yielding a 47% loss of starting material after 6 hours at 60°C. The products obtained from reactions of the amines, both alkyl and aromatic, were different from those obtained with the metal complex reactions. Based on its level of reactivity and looking toward other antisense oligonucleotides equipped with such reactive moieties, a more extensive analysis was conducted using triethylene tetramine.

To further define and locate the site(s) of reaction of triethylene tetramine upon the 5' Cap structure, three additional substrates were used: GpppG, GMP, and m7GMP. This reaction set allowed differentiation between chemistry occurring at the phosphoanhydride linkages vs. chemistry occurring at either of the guanine bases. After 12 hours at 60°C, 500 mM triethylene tetramine, 1 mM substrate, no reaction is observed with either GpppG or GMP, whereas 68% loss of m7GMP is observed under these conditions, a loss comparable to that of the complete cap substrate. These results indicate that the primary or initiating reaction center for triethylene tetramine is located solely on the N7-methylated guanosine residue, not the phosphoanhydride linkage nor the unmethylated guanosine residue.

In general, the sites of reactivity for the metal complexes upon the 5' Cap structure are different from those of the amines; the metal complexes that we have examined preferentially hydrolyze the triphosphoanhydride linkage, whereas the amines (exemplified by triethylene tetramine) preferentially react with the 7-methylguanosine residue. This is shown in Table 2:

**TABLE 2**

| | |
|---|---|
| (Sites of reactivity shown in bold:) | |

| Metal complexes: | Amines: |
|---|---|
| m7G**ppp**G | **m7G**pppG |

### Example 4

Chemical synthesis of oligonucleotides: Unmodified oligonucleotides were synthesized on an automated DNA synthesizer (Applied Biosystems model 380B) using standard phosphoramidate chemistry with oxidation by iodine.

### Example 5

Synthesis of 5' methylated guanosine (m7G)-capped oligoribonucleotides: Capped oligoribonucleotides may be prepared by ligating commercially available m7GpppG to a chemically synthesized, 5'phosphorylated oligoribonucleotide, using T4 RNA ligase (Pharmacia). 2.5 mM m7GpppG, 0.3 mM oligoribonucleotide, 3.0 mM ATP, 30 units T4 RNA ligase were combined in 50 mM HEPES, pH 8.0, plus 10 mM MgCl₂, 0.7% DMSO. Reaction was for 48 hours at 4°C. Ligation yield was approximately 50%.

### Example 6

Effect of 3' dangling bases on the hybridization properties of an antisense oligodeoxyribonucleotide complementary to a 5'-capped oligoribonucleotide: A 5'-capped oligonucleotide, 14 nucleotides in length exclusive of the cap, was synthesized by ligating commercially available m7GpppG (Pharmacia) to the chemically synthesized 5'-phosphorylated 14-mer, herein referred to as ISIS 2975 (see TABLE 3 below for sequence information), using T4 RNA ligase (Pharmacia). The desired product (50% ligation yield) was gel purified under native conditions and extracted into distilled water by crushing and soaking. Verification of capped product was done by comparison of the gel mobilities of the starting material (faster) and purified product (slower), and by the observation that the capped product (referred to herein as ISIS m7G2975) was insensitive to dephosphorylation with calf alkaline phosphatase (Boehringer Mannheim) while the 5'-phosphorylated starting material, ISIS 2975, was dephosphorylated under the same reaction conditions.

Two antisense oligonucleotides, ISIS 3043 and ISIS 3044, complementary to ISIS 2975 were synthesized. The sequences of these three oligonucleotides are shown in TABLE 3.

**TABLE 3**

| Ref. no. | Sequence (5'-3') | Seg. ID No. |
|---|---|---|
| ISIS-2975 | CUA UAA GGA UCA CG | 1 |
| ISIS-3044 | GTC ATC CTT ATA GC | 2 |
| ISIS-3043 | GTC ATC CTT ATA GCG C | 3 |

ISIS 3044, is a 14-mer complementary to ISIS 2975 base for base in the 14-base oligoribonucleotide region. ISIS 3043, a 16-mer, has two additional noncomplementary bases, G and C, at its 3' end (which upon specific hybridization with the capped ISIS m7G2975 would be opposite the cap structure). Each antisense oligonucleotide was mixed 1:1 with ISIS m7G2975 at a concentration of 3 µM in 100 mM sodium and 10mM phosphate (pH 7.0). Each sample was then heat denatured at 85°C for 5 minutes and slowly cooled to room temperature. Thermal melts were then conducted over a temperature range from 10°C to 90°C using a Gilford Response II at steps of 0.5°C. The Tm's were determined by taking the first derivative of the melt profiles. The thermal melt profiles for the ISIS m7G2975:ISIS 3044 duplex and the ISIS m7G2975:ISIS 3043 duplex were compared. For the ISIS 3043:ISIS m7G2975 duplex, the Tm was found to be 41.5°C; for the ISIS 3044:ISIS m7G2975 duplex, the Tm was found to be 42.5°C. These Tm's are identical within experimental error.

This result indicates that additional dangling bases at the 3' end of an antisense oligonucleotide directed to the 5' terminus of a capped RNA will not alter the hybridization properties of the oligonucleotide as a result of interactions between these dangling bases and the 5' cap structure of the transcript. These results indicate that attachment of a moiety which binds or reacts with the 5' Cap of mRNA to the 3' end of an antisense molecule should be possible without perturbing the hybridization properties of the antisense molecule to the RNA.

## Claims

1. A composition for modulating the activity of an RNA comprising:
a reactive portion capable of modifying or removing the 5' Cap structure of mRNA;
a targeting portion specifically hybridizable with the 5' terminal region of a targeted RNA for placement of the reative portion in a reactive position for the 5' Cap; and
a tether for connecting the targeting and reactive portions.

2. The composition of claim 1 adapted for placement of said reactive portion in a position to react with an atom, bond, bonds, or conformation of the 5' cap structure.

3. The composition of claim 1 wherein said reactive portion comprises a nucleophilic functionality capable of cleavage of the phosphoanhydride bond of the 5' Cap structure.

4. The composition of claim 1 wherein said reactive portion comprises a coordination complex of a metal.

5. The composition of claim 1 wherein said reactive portion comprises a moiety capable of stabilizing the transition state formed in cleavage of the phosphoanhydride bond of the 5' cap structure.

6. The composition of claim 1 wherein said reactive portion comprises an acidic functionality capable of catalyzing the cleavage of the phosphoanhydride bond of the 5' cap structure.

7. The composition of claim 1 wherein said reactive portion comprises an alkylating functionality.

8. The composition of claim 7 wherein the alkylating functionality is selected from the group consisting of sulfonyls, alkyl halides, alpha-halo carbonyls and amides, aziridine, nitrogen mustards, Michael acceptors and other relevant alkylating agents.

9. The composition of claim 1 wherein said reactive portion intercalates with the 5' cap structure and/or initial 5' most bases of the selected RNA.

10. The composition of claim 1 wherein said reactive portion comprises a functionality capable of forming free radicals.

11. The composition of claim 1 wherein said tether comprises from 1 to about 50 atoms.

12. The composition of claim 1 wherein said tether comprises from 1 to about 10 atoms.

13. The composition of claim 11 where the tether has at least one side chain group.

14. The composition of claim 13 wherein the side chain group is a heterocyclic base.

15. The composition of claim 14 wherein the base is a cytosine.

16. The composition of claim 13 wherein the side chain is a cationic functional group.

17. The composition of claim 16 wherein said functional group is guanidines, amidines, amines, or metal complexes.

18. The composition of claim 1 wherein said targeting portion is an oligonucleotide or oligonucleotide analog comprising from about 5 to about 50 base units.

19. The composition of claim 1 wherein said targeting portion is an oligonucleotide or oligonucleotide analog comprising from about 8 to 40 base units.

20. The composition of claim 1 wherein said targeting portion is an oligonucleotide or oligonucleotide analog comprising about 15 base units.

21. The composition of claim 1 wherein said targeting portion is an oligonucleotide or oligonucleotide analog which specifically hybridizes to the 5' end of the target mRNA transcript.

22. The composition of claim 1 wherein said targeting portion is an oligonucleotide or oligonucleotide analog which specifically hybridizes to the immature pre-mRNA in the nucleus.

23. The composition of claim 1 wherein said targeting portion is an oligonucleotide analog wherein at least some phosphodiester bonds of an oligonucleotide have been substituted by a non-ionic and non-chiral linkages.

24. A composition according to claim 1 for use in the treatment of a disease involving the undesired production of a protein.

25. The composition of claim 1 wherein said reactive portion comprises a reactive moiety capable of cleavage of the phosphoanhydride bond of the 5' Cap structure.

26. The composition of claim 1 wherein said reactive portion comprises a functionality capable of structurally or chemically modifying the 7-methylguanosine residue of the 5' Cap structure.

27. The composition of claim 1 wherein said reactive portion comprises one or more alkyl amine moieties.

28. The composition of claim 27 wherein said alkyl amine moieties are selected from the group consisting of 1,5,9-triazacyclododecane, diethylene triamine and triethylene tetramine.

29. The composition of claim 1 wherein said reactive portion comprises one or more aromatic amine moieties.

30. The composition of claim 29 wherein said aromatic amine moieties are selected from the group consisting of: imidazole, N-methylimidazole, histamine and pyridine.

31. The composition of claim 1 wherein said reactive moiety comprises an acidic functionality capable of cleaving the phosphoanhydride bond of the 5' Cap structure.

32. The composition of claim 1 wherein said reactive portion masks the 5' cap of RNA by inhibiting the binding of the 5' Cap-specific binding proteins.

33. The composition of claim 1 wherein said tether comprises from 1 to about 500 atoms.

34. The composition of claim 1 wherein said tether comprises at lease one nucleotide.

35. The composition of claim 1 wherein said tether comprises at least one amino acid.

36. The composition of claim 1 wherein said targeting portion is an oligonucleotide or oligonucleotide analog comprising from about 15 to about 25 base units.

37. The composition of claim 1 wherein said targeting portion is an oligonucleotide analog wherein at least one phosphodiester bond between nucleotides has been replaced by non-ionic and non-chiral linkages.

38. The composition of claim 1 wherein said targeting portion is an oligonucleotide analog having at least one phosphodiester bond is replaced with a sulfur-containing linkage.

39. The composition of claim 38 wherein said sulfur-containing linkage is a phosphorothioate moiety.

40. The composition of claim 1 wherein said targeting portion is an oligonucleotide analog having at least one phosphodiester bond substituted by an enantiomerically specific chiral linkage.

41. The composition of claim 1 wherein said targeting portion comprises at least one modified base.

42. The composition of claim 4 wherein said coordination complex of a metal is selected from the group consisting of: zinc(II) complex of 1,10-*ortho*-phenanthroline, zinc (II) complex of bipyridine, copper(II) complex of 1,10-*ortho*-phenanthroline, and copper (II) complex of bipyridine.

## Patentansprüche

1. Zusammensetzung zur Modulation der Aktivität einer RNA, die umfaßt:
einen reaktiven Anteil, der die 5' Cap-Struktur von mRNA modifizieren oder entfernen kann;
einen Targeting-Anteil, der mit der 5'-Terminal-Region einer Target-RNA spezifisch hybridisierbar ist zur Anordnung des reaktiven Anteils in einer reaktiven Position für die 5' Cap-Struktur; und
einen Tether zum Verbinden der Targeting- und reaktiven Anteile.

2. Zusammensetzung nach Anspruch 1, die geeignet ist für die Anordnung des genannten reaktiven Anteils in einer solchen Position, daß er mit einem Atom, einer Bindung, mehreren Bindungen oder einer Konformation der 5' cap-Struktur reagiert.

3. Zusammensetzung nach Anspruch 1, in der der genannte reaktive Abschnitt eine nucleophile Funktionalität umfaßt, die in der Lage ist, die Phosphoanhydrid-Bindung der 5' Cap-Struktur zu schneiden (zu spalten).

4. Zusammensetzung nach Anspruch 1, in der der genannte reaktive Anteil einen Koordinationskomplex eines Metalls umfaßt.

5. Zusammensetzung nach Anspruch 1, in der der genannte reaktive Anteil einen Rest umfaßt, der in der Lage ist, den beim Schneiden (Spalten) der Phosphoanhydrid-Bindung der 5' cap-Struktur gebildeten Übergangszustand zu stabilisieren.

6. Zusammensetzung nach Anspruch 1, in der der genannte reaktive Anteil eine saure Funktionalität umfaßt, die in der Lage ist, das Schneiden (Spalten) der Phosphoanhydrid-Bindung der 5' cap-Struktur zu katalysieren.

7. Zusammensetzung nach Anspruch 1, in der der genannte reaktive Anteil eine Alkylierungs-Funktionalität umfaßt.

8. Zusammensetzung nach Anspruch 7, in der die Alkylierungs-Funktionalität ausgewählt wird aus der Gruppe, die besteht aus Sulfonylen, Alkylhalogeniden, α-Halogencarbonylen und Amiden, Aziridin, Stickstoff-Senfen, Michael-Akzeptoren und anderen relevanten Alkylierungsmitteln.

9. Zusammensetzung nach Anspruch 1, in der der genannte reaktive Anteil intercaliert mit der 5' cap-Struktur und/oder den meisten Anfangs- 5'-Basen der ausgewählten RNA.

10. Zusammensetzung nach Anspruch 1, in der der genannte reaktive Anteil eine Funktionalität umfaßt, die freie Radikale bilden kann.

11. Zusammensetzung nach Anspruch 1, in der der genannte Tether 1 bis etwa 50 Atome umfaßt.

12. Zusammensetzung nach Anspruch 1, in der der genannte Tether 1 bis etwa 10 Atome umfaßt.

13. Zusammensetzung nach Anspruch 11, in der der Tether mindestens eine Seitenkettengruppe aufweist.

14. Zusammensetzung nach Anspruch 13, in der die Seitenkettengruppe eine heterocyclische Base ist.

15. Zusammensetzung nach Anspruch 14, in der die Base ein Cytosin ist.

16. Zusammensetzung nach Anspruch 13, in der die Seitenkette eine kationische funktionelle Gruppe ist.

17. Zusammensetzung nach Anspruch 16, in der die genannte funktionelle Gruppe ausgewählt wird aus Guanidinen, Amidinen, Aminen oder Metallkomplexen.

18. Zusammensetzung nach Anspruch 1, in der der genannte Targeting-Anteil ein Oligonucleotid oder Oligonucleotid-Analogon ist, das etwa 5 bis etwa 50 Basen-Einheiten umfaßt.

19. Zusammensetzung nach Anspruch 1, in der der genannte Targeting-Anteil ein Oligonucleotid oder Oligonucleotid-Analogon ist, das etwa 8 bis 40 Basen-Einheiten umfaßt.

20. Zusammensetzung nach Anspruch 1, in der der genannte Targeting-Anteil ein Oligonucleotid oder Oligonucleotid-Analogon ist, das etwa 15 Basen-Einheiten umfaßt.

21. Zusammensetzung nach Anspruch 1, in der der genannte Targeting-Anteil ein Oligonucleotid oder Oligonucleotid-Analogon ist, das spezifisch hybridisiert mit dem 5'-Ende des Target-mRNA-Transkripts.

22. Zusammensetzung nach Anspruch 1, in der der genannte Targeting-Anteil ein Oligonucleotid oder Oligonucleotid-Analogon ist, das mit der unreifen pre-mRNA in dem Kern spezifisch hybridisiert.

23. Zusammensetzung nach Anspruch 1, in der der genannte Targeting-Anteil ein Oligonucleotid-Analogon ist, in dem mindestens einige Phosphodiester-Bindungen eines Oligonucleotids durch nicht-ionische und nicht-chirale Verknüpfungen ersetzt worden sind.

24. Zusammensetzung nach Anspruch 1 für die Verwendung bei der Behandlung einer Erkrankung, welche die unerwünschte Bildung eines Proteins umfaßt.

25. Zusammensetzung nach Anspruch 1, in der der genannte reaktive Anteil einen reaktiven Rest umfaßt, der in der Lage ist, die Phosphoanhydrid-Bindung der 5' Cap-Struktur zu schneiden (zu spalten).

26. Zusammensetzung nach Anspruch 1, in der der genannte reaktive Anteil eine Funktionalität umfaßt, die in der Lage ist, den 7-Methylguanosin-Rest der 5' Cap-Struktur strukturell oder chemisch zu modifizieren.

27. Zusammensetzung nach Anspruch 1, in der der genannte reaktive Anteil einen oder mehr Alkylamin-Reste umfaßt.

28. Zusammensetzung nach Anspruch 27, in der die genannten Alkylamin-Reste ausgewählt werden aus der Gruppe, die besteht aus 1,5,9-Triazacyclododecan, Diethylentriamin und Triethylentetramin.

29. Zusammensetzung nach Anspruch 1, in der der genannte reaktive Anteil einen oder mehr aromatische Amin-Reste umfaßt.

30. Zusammensetzung nach Anspruch 29, in der die genannten aromatischen Amin-Reste ausgewählt werde aus der Gruppe, die besteht aus Imidazol, N-Methylimidazol, Histamin und Pyridin.

31. Zusammensetzung nach Anspruch 1, in der der genannte reaktive Rest eine saure Funktionalität umfaßt, welche die Phosphoanhydrid-Bindung der 5' Cap-Struktur schneiden (spalten) kann.

32. Zusammensetzung nach Anspruch 1, in der der genannte reaktive Anteil das 5' Cap der RNA maskiert durch Inhibierung der Bindung der 5' Capspezifischen Bindungs-Proteine.

33. Zusammensetzung nach Anspruch 1, in der der genannte Tether 1 bis etwa 500 Atome umfaßt.

34. Zusammensetzung nach Anspruch 1, in der der genannte Tether mindestens ein Nucleotid umfaßt.

35. Zusammensetzung nach Anspruch 1, in der der genannte Tether mindestens eine Aminosäure umfaßt.

36. Zusammensetzung nach Anspruch 1, in der der genannte Targeting-Anteil ein Oligonucleotid oder Oligonucleotid-Analogon ist, das etwa 15 bis etwa 25 Basen-Einheiten umfaßt.

37. Zusammensetzung nach Anspruch 1, in der der genannte TargetingAnteil ein Oligonucleotid-Analogon ist, in dem mindestens eine Phosphodiester-Bindung zwischen Nucleotiden durch nicht-ionische und nicht-chirale Verknüpfungen ersetzt worden ist.

38. Zusammensetzung nach Anspruch 1, in der der genannte TargetingAnteil ein Oligonucleotid-Analogon ist, das mindestens eine Phosphodiester-Bindung aufweist, die durch eine Schwefel enthaltende Verknüpfung ersetzt ist.

39. Zusammensetzung nach Anspruch 38, in der die genannte Schwefel enthaltende Verknüpfung ein Phosphorothioat-Rest ist.

40. Zusammensetzung nach Anspruch 1, in der der genannte Targeting-Anteil ein Oligonucleotid-Analogon ist, das mindestens eine Phosphodiester-Bindung aufweist, die durch eine Enantiomer-spezifische chirale Verknüpfung substituiert ist.

41. Zusammensetzung nach Anspruch 1, in der der genannte Targeting-Anteil mindestens eine modifizierte Base umfaßt.

42. Zusammensetzung nach Anspruch 4, in der der genannten Koordinationskomplex eines Metalls ausgewählt wird aus der Gruppe, die besteht aus dem Zink(II)-Komplex von 1,10-ortho-Phenanthrolin, dem Zink(II)-Komplex von Bipyridin, dem Kuper(II)-Komplex von 1,10-ortho-Phenanthrolin und dem Kupfer(II)-Komplex von Bipyridin.

## Revendications

1. Composition pour moduler l'activité d'un ARN comprenant :
une partie réactive capable de modifier ou d'enlever la structure de la coiffe en 5' de l'ARNm;
une partie de ciblage capable de s'hybrider spécifiquement avec la région terminale en 5' d'un ARN ciblé pour permettre la mise en place de la partie réactive dans une position réactive vis-à-vis de la coiffe en 5'; et
un lien servant à relier la partie de ciblage et la partie réactive.

2. Composition selon la revendication 1 conçue pour la mise en place de ladite partie réactive dans une position lui permettant de réagir avec un atome, une liaison, des liaisons, ou une conformation de la structure de la coiffe en 5'.

3. Composition selon la revendication 1 dans laquelle ladite partie réactive comprend une fonctionnalité nucléophile capable de couper la liaison phosphoanhydride de la structure de la coiffe en 5'.

4. Composition selon la revendication 1 dans laquelle ladite partie réactive comprend un complexe de coordination d'un métal.

5. Composition selon la revendication 1 dans laquelle ladite partie réactive comprend un fragment capable de stabiliser l'état de transition formé lors de la coupure de la liaison phosphoanhydride de la structure de la coiffe en 5'.

6. Composition selon la revendication 1 dans laquelle ladite partie réactive comprend une fonctionnalité acide capable de catalyser la coupure de la liaison phosphoanhydride de la structure de la coiffe en 5'.

7. Composition selon la revendication 1 dans laquelle ladite partie réactive comprend une fonctionnalité alkylante.

8. Composition selon la revendication 7 dans laquelle la fonctionnalité alkylante est choisie dans le groupe comprenant les sulfonyles, les halogénures d'alkyle, les alpha-halogéno-carbonyles et amides, l'aziridine, les moutardes azotées, les accepteurs de Michael et d'autres agents alkylants pertinents.

9. Composition selon la revendication 1 dans laquelle ladite partie réactive s'intercale dans la structure de la coiffe en 5' et/ou la plupart des bases en 5' initiales de l'ARN sélectionné.

10. Composition selon la revendication 1 dans laquelle ladite partie réactive comprend une fonctionnalité capable de former des radicaux libres.

11. Composition selon la revendication 1 dans laquelle ledit lien comprend de 1 à environ 50 atomes.

12. Composition selon la revendication 1 dans laquelle ledit lien comprend de 1 à environ 10 atomes.

13. Composition selon la revendication 11 dans laquelle ledit lien possède au moins un groupe à chaîne latérale.

14. Composition selon la revendication 13 dans laquelle le groupe à chaîne latérale est une base hétérocyclique.

15. Composition selon la revendication 14 dans laquelle la base est une cytosine.

16. Composition selon la revendication 13 dans laquelle la chaîne latérale est un groupe fonctionnel cationique.

17. Composition selon la revendication 16 dans laquelle ledit groupe fonctionnel consiste en guanidines, amidines, amines, ou complexes métalliques.

18. Composition selon la revendication 1 dans laquelle ladite partie de ciblage est un oligonucléotide ou un analogue d'oligonucléotide comprenant d'environ 5 à environ 50 unités de base.

19. Composition selon la revendication 1 dans laquelle ladite partie de ciblage est un oligonucléotide ou un analogue d'oligonucléotide comprenant d'environ 8 à 40 unités de base.

20. Composition selon la revendication 1 dans laquelle ladite partie de ciblage est un oligonucléotide ou un analogue d'oligonucléotide comprenant environ 15 unités de base.

21. Composition selon la revendication 1 dans laquelle ladite partie de ciblage est un oligonucléotide ou un analogue d'oligonucléotide qui s'hybride spécifiquement à l'extrémité 5' du transcrit d'ARNm-cible.

22. Composition selon la revendication 1 dans laquelle ladite partie de ciblage est un oligonucléotide ou un analogue d'oligonucléotide qui s'hybride spécifiquement au pré-ARNm immature dans le noyau.

23. Composition selon la revendication, 1 dans laquelle ladite partie de ciblage est un analogue d'oligonucléotide dans lequel au moins quelques-unes des liaisons phosphodiesters d'un oligonucléotide ont été remplacées par des chaînons non ioniques et non chiraux.

24. Composition selon la revendication 1 à utiliser dans le traitement d'une maladie impliquaut la production indésirable d'une protéine.

25. Composition selon la revendication 1 dans laquelle ladite partie réactive comprend un fragment réactif capable de couper la liaison phosphoanhydride de la structure de la coiffe en 5'.

26. Composition selon la revendication 1 dans laquelle ladite partie réactive comprend une fonctionnalité capable de modifier structurellement ou chimiquement le résidu de 7-méthylguanosine de la structure de la coiffe en 5'.

27. Composition selon la revendication 1 dans laquelle ladite partie réactive comprend un ou plusieurs fragments alkylamines.

28. Composition selon la revendication 27 dans laquelle lesdits fragments alkylamines sont choisis dans le groupe constitué du 1,5,9-triazacyclododécane, de la diéthylènetriamine et de la triéthylènetétramine.

29. Composition selon la revendication 1 dans laquelle ladite partie réactive comprend un ou plusieurs fragments amines aromatiques.

30. Composition selon la revendication 29 dans laquelle lesdits fragments amines aromatiques sont choisis dans le groupe constitué de l'imidazole, du N-méthylimidazole, de l'histamine et de la pyridine.

31. Composition selon la revendication 1 dans laquelle ladite partie réactive comprend une fonctionnalité acide capable de couper la liaison phosphoanhydride de la structure de la coiffe en 5'.

32. Composition selon la revendication 1 dans laquelle ladite partie réactive masque la coiffe en 5' de l'ARN en inhibant la liaison des protéines de liaison spécifiques de la coiffe en 5'.

33. Composition selon la revendication 1 dans laquelle ledit lien comprend de 1 à environ 500 atomes.

34. Composition selon la revendication 1 dans laquelle ledit lien comprend au moins un nucléotide.

35. Composition selon la revendication 1 dans laquelle ledit lien comprend au moins un acide aminé.

36. Composition selon la revendication 1 dans laquelle ladite partie de ciblage est un oligonucléotide ou un analogue d'oligonucléotide comprenant d'environ 15 à environ 25 unités de base.

37. Composition selon la revendication 1 dans laquelle ladite partie de ciblage est un analogue d'oligonucléotide dans lequel au moins une liaison phosphodiester entre des nucléotides a été remplacée par des chaînons non ioniques et non chiraux.

38. Composition selon la revendication 1 dans laquelle ladite partie de ciblage est un analogue d'oligonucléotide ayant au moins une liaison phosphodiester remplacée par un chaînon soufré.

39. Composition selon la revendication 38 dans laquelle ledit chaînon soufré est un fragment phosphorothioate.

40. Composition selon la revendication 1 dans laquelle ladite partie de ciblage est un analogue d'oligonucléotide ayant au moins une liaison phosphodiester remplacée par un chaînon chiral énantiomériquement spécifique.

41. Composition selon la revendication 1 dans laquelle ladite partie de ciblage comprend au moins une base modifiée.

42. Composition selon la revendication 4 dans laquelle ledit complexe de coordination d'un métal est choisi dans le groupe constitué du complexe de zinc(II) de la 1,10-*ortho*-phénanthroline, du complexe de zinc(II) de la bipyridine, du complexe de cuivre(II) de la 1,10-*ortho*-phénanthroline, et du complexe de cuivre(II) de la bipyridine.


SEQUENCE LISTING

(1) GENERAL INFORMATION:

(i) APPLICANT: ISIS Pharmaceuticals and
Brenda Baker

(ii) TITLE OF INVENTION: Compositions and Methods for Modulating
RNA Activity Through Modification of the 5' Cap Structure
of RNA

(iii) NUMBER OF SEQUENCES: 3

(iv) CORRESPONDENCE ADDRESS:

(A) ADDRESSEE: Woodcock Washburn Kurtz Mackiewicz & Norris

(B) STREET: One Liberty Place - 46th Floor

(C) CITY: Philadelphia

(D) STATE: PA

(E) COUNTRY: USA

(F) ZIP: 19103

(v) COMPUTER READABLE FORM:

(A) MEDIUM TYPE: DISKETTE, 3.5 INCH, 1.44 Mb STORAGE

(B) COMPUTER: IBM PS/2

(C) OPERATING SYSTEM: PC-DOS

(D) SOFTWARE: WORDPERFECT 5.0

(vi) CURRENT APPLICATION DATA:

(A) APPLICATION NUMBER:n/a

(B) FILING DATE: herewith

(C) CLASSIFICATION:

(vii) PRIOR APPLICATION DATA:

(A) APPLICATION NUMBER: 527,599

(B) FILING DATE: May 23, 1990

(viii) ATTORNEY/AGENT INFORMATION:

(A) NAME: Jane Massey Licata
(B) REGISTRATION NUMBER: 32,257
(C) REFERENCE/DOCKET NUMBER: ISIS-0256
(ix) TELECOMMUNICATION INFORMATION:
(A) TELEPHONE: (215) 568-3100
(B) TELEFAX: (215) 568-3439

(2) INFORMATION FOR SEQ ID NO: 1:
(i) SEQUENCE CHARACTERISTICS:
(A) LENGTH: 14
(B) TYPE: nucleic acid
(C) STRANDEDNESS: single
(D) TOPOLOGY: unknown
(xi) SEQUENCE DESCRIPTION: SEQ ID NO: 1:
CUAUAAGGAU CACG (14)

(2) INFORMATION FOR SEQ ID NO: 2:
(i) SEQUENCE CHARACTERISTICS:
(A) LENGTH: 14
(B) TYPE: nucleic acid
(C) STRANDEDNESS: single
(D) TOPOLOGY: unknown
(xi) SEQUENCE DESCRIPTION: SEQ ID NO: 2:
GTCATCCTTA TAGC (14)

(2) INFORMATION FOR SEQ ID NO: 3:
(i) SEQUENCE CHARACTERISTICS:
(A) LENGTH: 16
(B) TYPE: nucleic acid
(C) STRANDEDNESS: single
(D) TOPOLOGY: unknown
(xi) SEQUENCE DESCRIPTION: SEQ ID NO: 3:
GTCATCCTTA TAGCGC (16)